# FASCICULE DE BREVET EUROPEEN

(11) **EP 0 612 763 B1**
(45) Date de publication et mention de la délivrance du brevet: **14.05.1997**
(21) Numéro de dépôt: 94400346.6
(22) Date de dépôt: 17.02.1994
(51) Int. Cl.: C07K 14/655, A61K 38/31

(54) **Dérivés cyclopeptidiques de l'angiopeptine, leur procédé de préparation et les compositions pharmaceutiques qui les contiennent**
Zyklische Angiopeptin Derivate, deren Herstellung und pharmazeutische Zusammensetzungen
Cyclic derivatives of angiopeptin, their preparation and their pharmaceutical compositions

(30) Priorité: 22.02.1993 FR 9301978
(43) Date de publication de la demande: 31.08.1994
(73) Titulaire: ADIR ET COMPAGNIE, 92415 Courbevoie Cédex (FR)
(72) Inventeur: Fauchere, Jean-Luc, F-92210 Saint-Cloud (FR); Thurieau, Christophe, F-75116 Paris (FR); Vilaine, Jean-Paul, F-92290 Chatenay Malabry (FR); Janiak, Philip, F-78810 Faucherolles (FR)

(56) Documents cités:
- EP-A- 0 203 031
- WO-A-89/12068

## Description

La présente invention concerne de nouveaux dérivés peptidiques de l'angiopeptine, leur procédé de préparation et les compositions pharmaceutiques qui les contiennent.

L'angiopeptine est un octapeptide amide contenant les acides aminés non naturels 3-(2-naphtyl)-_{D}-alanine (_{D}-Nal) et _{D}-tryptophane (_{D}-Trp). Cet octapeptide est partiellement cyclique, un pont disulfure relie les deux chaînes latérales des cystéines en position 2 et 7 ; il répond à la formule : 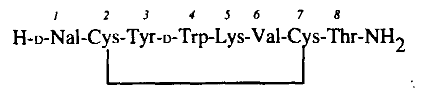 Lundergan et al. (Atherosclerosis, 80, 49-55, (1989)) ont décrit pour l'angiopeptine un effet antiprolifératif in vivo sur des cellules du muscle lisse vasculaire dans un modèle d'artère carotide du rat impliquant une dessication à l'air de l'endothélium.

L'art antérieur est illustré par les brevets EP-A-0 203 301 et WO-A-89/12068 qui décrivent des octapeptides cycliques contenant un résidu lysine.

La demanderesse a découvert que les propriétés pharmacologiques de l'angiopeptine pouvaient être grandement améliorées grâce aux deux modifications structurales suivantes :
1) - remplacement du pont disulfure par une liaison amide.
2) - optimisation de la taille du cycle grâce au choix de longueur correcte des chaînes latérales des résidus d'acides aminés sur lesquels s'effectue la cyclisation.
Le remplacement du pont disulfure par une liaison amide a été guidé par les différences structurales fondamentales de ces deux types de cyclisation :

| Pont disulfure | Liaison amide |
|---|---|
| - non planaire | - planaire |
| - libre rotation autour de la liaison sigma | - position relative "trans" des substituants |
| - liaisons hydrogène impossibles | - liaisons hydrogène possibles avec d'autres accepeurs ou donneurs |

La taille du cycle entre les positions 2 et 7 est de nature à modifier considérablement sa conformation ainsi que l'orientation des résidus 1 et 8 à l'extérieur du cycle.
La demanderesse a découvert que les composés possédant l'effet inhibiteur optimal vis à vis d'une lésion vasculaire proliférative sont ceux dont le cycle admet 22 atomes.
Les dérivés de l'invention possèdent donc des propriétés pharmacologiques supérieures aux analogues décrits ou revendiqués à ce jour. Notamment, ils inhibent la prolifération myointimale de la carotide de rat *in vivo* de façon plus efficace que l'angiopeptine ou autres analogues connus.

L'invention concerne plus particulièrement de nouveaux dérivés peptidiques répondant à la formule générale (I) : 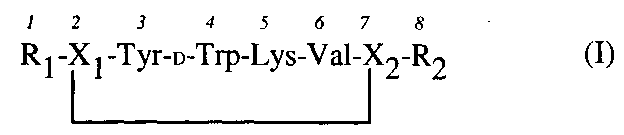 dans laquelle :
- R₁ représente un résidu d'acide aminé de configuration L ou D, acétylé ou non, communément utilisé par l'homme de l'art,
- R₂ représente un résidu d'acide aminé de configuration L ou D, communément utilisé par l'homme de l'art et possédant une fonction C-terminale amide ou acide libre,
- X₁ et X₂ sont choisis parmi des résidus d'acides aminés permettant une cyclisation par une liaison amide et conférant au cycle ainsi formé un nombre d'atomes égal à 22,
- Tyr représente le résidu _{L}-Tyrosyle,
- _{D}-Trp représente le résidu _{D}-Tryptophyle,
- Lys représente le résidu _{L}-Lysyle,
- Val représente le résidu _{L}-Valyle,
leurs énantiomères, diastéréoisomères et épimères, ainsi que leurs sels d'addition à un acide ou à une base pharmaceutiquement acceptable.

Le terme résidu d'acide aminé représente un acide aminé auquel il manque un atome d'hydrogène sur la fonction amine, ou le groupement hydroxyle sur la fonction acide, ou bien auquel il manque à la fois un atome d'hydrogène sur la fonction amine et le groupement hydroxyle de la fonction acide.
D'une manière générale, les règles de nomenclature peptidique utilisées dans la présente invention sont conformes aux recommandations de 1983 de l'IUPAC-IUB (Eur. J. Biochem., 138, 9-36, (1984)).

Plus précisément, l'invention concerne les composés de formule générale (I) dans laquelle :
- R₁ représente un résidu d'acide aminé de configuration L ou D, dont l'amine terminale peut être acétylée ou non et choisi parmi l'alanine, la cystéine, la glycine, l'histidine, la leucine, l'acide glutamique, la glutamine, l'arginine, l'asparagine, l'acide aspartique, la lysine, la méthionine, la phénylalanine, la proline, la sérine, la thréonine, le tryptophane, la tyrosine, la valine et la naphtylalanine,
- R₂ représente un résidu d'acide aminé de configuration L ou D, possédant une fonction C-terminale amide ou alcool, acide libre, l'acide aminé étant choisi parmi l'alanine, méthylalanine, la phénylalanine, la naphtylalanine, l'arginine, l'aspargine, l'acide aspartique, la la cystéine, la glutamine, l'acide glutamique, la glycine, l'histidine, la leucine, la lysine, la méthionine, la proline, l'hydroxyproline, la sérine, la thréonine, le tryptophane, la tyrosine et la valine,
- X₁ et X₂ sont choisis de manière à ce que le cycle des composés de la formule générale (I) contienne 22 atomes, ils sont **dépendants** et sont choisis parmi les résidus d'acides aminés suivants :

| **X**_{**1**} | **X**_{**2**} |
|---|---|
| -Glu- | -Dab- |
| -Asp- | -Orn- |
| -*homo* Glu- | -Dpr- |
| -Dab- | -Glu- |
| -Orn- | -Asp- |
| -Dpr- | -*homo* Glu- |

-Dab- représente le résidu de l'acide _{L}-diaminobutyrique et -Dpr-, le résidu de l'acide _{L}-diaminopropionique,
leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, camphorique, etc...

Parmi les bases pharmaceutiquement acceptables, on peut citer à titre non limitatif l'hydroxyde de sodium, l'hydroxyde de potassium, la tributylamine, la tertbutylamine, etc...

L'invention s'étend aussi au procédé de préparation des dérivés de formule (I) caractérisé en ce qu'ils peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B. W. Erickson et R. B. Merrifield ("The proteins", Solid-Phase Peptide Synthesis, 3rd edition, Volume II, 257-527, (1976)).

La synthèse sur phase solide peut se réaliser sur un automate qui effectue de façon répétitive et programmable des cycles de déprotections, couplages et lavages nécessaires à l'introduction séquentielle des acides aminés dans la chaîne peptidique. L'acide aminé, de préférence C-terminal, est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la fixation d'une fonction C-terminale, acide carboxylique, amide ou alcool.

Le choix du site de couplage des fragments est souvent déterminé de façon à minimiser les risques de racémisation. Trois sites possibles de couplage non-racémisants sont, par exemple, la fonction C-terminale de la 2-proline, de la 3-hydroxyproline et de la 4-glycine.

Les acides aminés sont alors introduits un à un dans l'ordre déterminé par l'opérateur. Chaque cycle de synthèse correspondant à l'introduction d'un acide aminé comporte une déprotection, de préférence N-terminale de la chaîne peptidique, des lavages successifs destinés à éliminer les réactifs, un couplage avec activation de l'acide aminé et de nouveaux lavages. Chacune de ces opérations est suivie d'une filtration réalisée sur verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBt) ou le benzotriazol-1-yl-oxytris(diméthylamino)phosphonium hexafluorophosphate (BOP) ou encore le diphénylphosphorylazide (DPPA). Des activations par formation d'anhydrides mixtes sont également possibles.

Chaque acide aminé est introduit dans le réacteur en excès, par exemple quadruple excès, par rapport au degré de substitution de la résine et en quantité environ équivalente par rapport aux agents de couplage. La réaction de couplage peut être vérifiée à chaque étape de la synthèse par le test de réaction à la ninhydrine décrit par E. Kaiser et coll. (Analyt. Biochem., 34, 595-599, (1970)).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole permet de séparer le peptide de la résine et de libérer éventuellement le peptide de ses groupes protecteurs. Le composé est alors purifié par des techniques classiques de purification, notamment chromatographiques. La cyclisation du peptide est ensuite réalisée en présence de BOP et de DIPEA.

Les peptides de la présente invention peuvent être également obtenus par couplage en solution de fragments peptidiques sélectivement protégés, qui peuvent être préparés soit sur phase solide, soit en solution. Les méthodes générales de la synthèse des peptides en solution sont, par exemple, décrites par F. M. Finn et K. Hofmann ("The Proteins", 3rd edition, Volume II, 105-253, (1976), "Synthesis of peptides by solution methods"). L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

Les composés de l'invention possèdent des propriétés pharmacologiques très intéressantes. Les études réalisées sur les produits de la présente invention montrent que ceux-ci permettent de prévenir efficacement in vivo le développement d'une lésion vasculaire sténosante notamment par une activité inhibitrice sur la composante de prolifération des cellules musculaires lisses vasculaires.

Ces propriétés permettent l'utilisation des composés de la présente invention comme médicament dans le traitement et la prévention des lésions vasculaires athéroscléreuses et en particulier pour prévenir les resténoses vasculaires après pontage, dilatation vasculaire, notamment coronaire, ou autres formes de reperméabilisation vasculaire, et après transplantation cardiaque.

Les produits de l'invention peuvent être également particulièrement utiles pour prévenir et traiter les altérations vasculaires liées à l'hypertension artérielle ou au diabète et notamment les atteintes rétiniennes.

L'activité antiprolifératrice des composés peut par ailleurs être utilisée dans le traitement de certains cancers et de certaines maladies dermatologiques comme le psoriasis.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols.

La posologie varie selon l'âge et le poids du patient, la nature et la sévérité de l'affection ainsi que la voie d'administration.

Celle-ci peut être orale, nasale, rectale ou parenterale. D'une manière générale, elle s'échelonne entre 0,2 et 100 mg pour un traitement en une ou plusieurs prises par 24 heures.

Les exemples suivants illustrent l'invention et ne la limitent en aucune façon.

### EXEMPLE 1 :

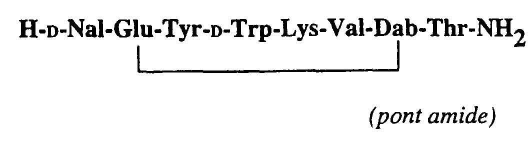

2 g d'une résine amide substituée à 0,5 mmoles/g de résine sont traités par la pipéridine à 20% dans le diméthylformamide (DMF). La synthèse proprement dite est alors effectuée selon le protocole répétitif suivant :

| **N° opération** | **Fonction** | **Solvant/Réactif** | **Répétition/temps** |
|---|---|---|---|
| 1 | lavage | DMF | 2 x 2 min |
| 2 | déprotection | 20% pipéridine/DMF | 1 x 5 min |
| 3 | déprotection | 20% pipéridine/DMF | 1 x 15 min |
| 4 | lavage | DMF | 3 x 2 min |
| 5 | lavage | dichlorométhane | 3 x 2 min |
| 6 | couplage | acide aminé protégé activé | 1 x 90 min |
| 7 | lavage | DMF | 3 x 2 min |
| 8 | lavage | alcool isopropylique | 3 x 2 min |
| 9 | lavage | dichlorométhane | 3 x 2 min |

Les acides aminés protégés ont été introduits dans l'ordre suivant :
Fmoc-Thr(But)-OH, Fmoc-Dbu(Boc)-OH, Fmoc-Val-OH,
Fmoc-Lys(Z)-OH, Fmoc-_{D}-Trp-OH, Fmoc-Tyr(But)-OH, Fmoc-Glu(OBut)-OH, Fmoc-_{D}-Nal-OH.

Les couplages sont effectués par dissolution de 4 équivalents de l'acide aminé protégé, avec 4 équivalents de HOBt et 4,4 équivalents de DCC. Les solvants utilisés sont 30 ml de DMF et 10 ml de dichlorométhane.

A la fin des huit cycles correspondant à la fixation séquentielle des huit acides aminés, la résine est traitée par un mélange d'acide trifluoroacétique (18 ml), éthanedithiol (1 ml) et anisole 1 ml, pendant 4 heures à 30°C. Le filtrat et les solvants de lavage de la résine sont réunis et évaporés à sec. Le produit obtenu, de formule : 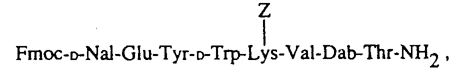 est précipité dans l'éther.

La cyclisation du peptide est ensuite réalisée en solution dans le DMF, en présence de BOP et de DIPEA. Le mélange est laissé sous agitation pendant 12 heures à température ambiante. Après évaporation du DMF, l'huile obtenue est purifiée sur colonne LH20. Le produit cyclisé ainsi obtenu : 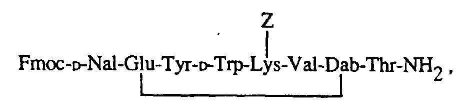 est traité par la pipéridine à 20% dans le DMF puis soumis à une hydrogénation catalytique dans un mélange de méthanol/eau (3/1). Le produit attendu est obtenu après purification par HPLC préparative sur phase inverse (colonne C₁₈ ; d = 47 mm, longueur 300 mm) puis lyophilisation.

L'analyse du peptide est réalisée après décomposition de celui-ci en acides aminés par hydrolyse dans de l'acide chlorhydrique 6N pendant 18 heures à 110°C et dosage quantitatif des acides aminés par HPLC.

| Compositions en acide aminés : | | | | | | |
|---|---|---|---|---|---|---|
| | **Glu** | **Thr** | **Val** | _{**D**}**-Nal** | **Lys** | **Tyr** |
| Calculé | 1 | 1 | 1 | 1 | 1 | 1 |
| Trouvé | 1.04 | 0.97 | 0.97 | 0.95 | 1.06 | 1.02 |

**Dab** : non déterminé, _{**D**}**-Trp** : non déterminé

Spectre de masse (FAB) : MH⁺: m/z = 1103.

Les exemples suivants sont réalisés selon le même procédé que celui décrit dans l'exemple 1.

### EXEMPLE 2 :

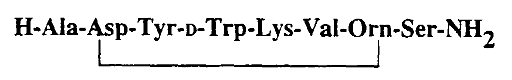

### EXEMPLE 3 :

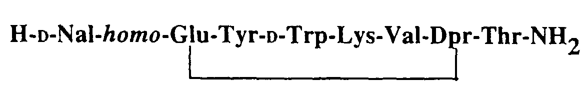

### EXEMPLE 4 :

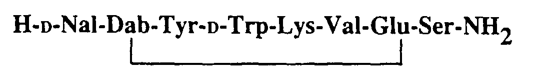

### EXEMPLE 5 :

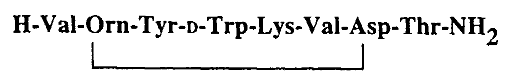

### EXEMPLE 6 :

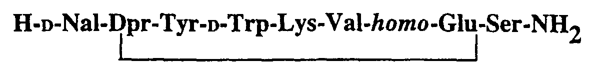

### ETUDE PHARMACOLOGIQUE DES COMPOSES DE L'INVENTION

### EXEMPLE 7 : Effets sur la prolifération myointimale induite par dénudation endothéliale.

Des rats Wistar mâles (450-500g, Charles River) sont prétraités par voie sous cutanée pendant les 2 jours précédant la dénudation endothéliale avec le composé de l'exemple 1 (100 µg/kg/jour), ou son véhicule. La dose journalière est administrée en deux fois. Après 2 jours de prétraitement, la carotide gauche commune est désendothélialisée par 3 passages successifs d'une sonde à embolectomie (Fogarty 2F, Baxter) et le traitement est poursuivi pendant 5 jours. Quatorze jours après la dénudation endothéliale, les rats sont anesthésiés au pentobartital (60 mg/kg ip) et les carotides sont fixées sous pression (11996 Pa) avant d'être prélevées. La partie centrale de chaque artère est divisée en 4 segments qui sont inclus en paraffine. Des coupes transversales de 5 µm non sériées sont ensuite réalisées sur chaque segment et colorées à l'orcéine. Les surfaces de la média et de la néointima sont mesurées par analyse d'images (Logiciel Histo, Biocom, Les Ulis). Les résultats sont reproduits dans le tableau I suivant :

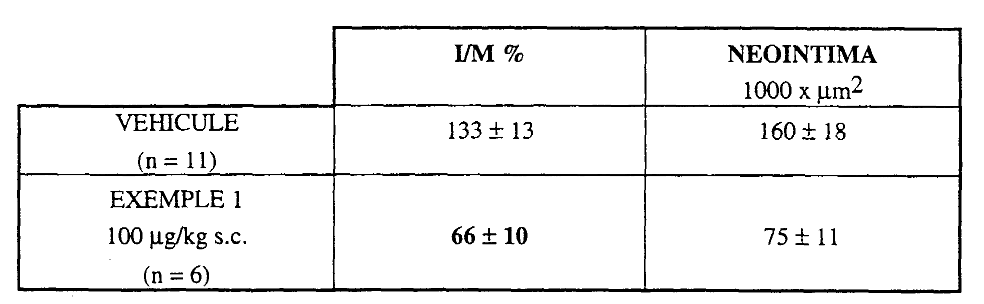

Durée du traitement : de -2 à +5 jours après la lésion. (Durée totale : 7 jours)
Mesures effectuées 14 jours après la lésion sur la carotide de rat
n = nombre de rats
I/M % : rapport Néointima/Média.

### EXEMPLE 8 : Effets sur l'incorporation de [³H]-Thymidine

Des rats Wistar mâles de 300g (Charles River) sont prétraités par voie sous cutanée pendant les 2 jours précédant la dénudation endothéliale avec le composé de l'exemple 1 (100 µg/kg/jour), ou son véhicule. La dose journalière est administrée en 2 fois. Après 2 jours de prétraitement, les animaux sont désendothélialisés selon la méthode suivante. Après anesthésie au méthohexital (Brietal, 60 mg/kg ip), une sonde à embolectomie (Fogarty 2F, Baxter) est introduite dans l'aorte par voie carotidienne. La désendothélialisation aortique est réalisée par trois passages successifs de la sonde à ballonnet. Le traitement est poursuivi pendant 3 jours.

Trois jours après la dénudation endothéliale, les rats sont sacrifiés et l'aorte est prélevée pour la détermination de l'incorporation de [³H]-thymidine. L'aorte thoracique est nettoyée des tissus adipeux adjacents dans du Krebs-Henseleit de composition suivante (en mmoles/l): NaCl: 120; KCl: 4,8; CaCl₂: 1,8; KH₂PO₄: 1,4; MgSO₄: 1,2; NaHCO₃: 25; glucose : 5, et sérum albumine bovine : 10 g/l; pH: 7,4, carbogène. La préparation vasculaire est ensuite préincubée à 37°C pendant une heure dans cette solution physiologique, puis transférée dans un Krebs-Henseleit de même composition mais enrichi en [³H]-thymidine (activité spécifique: 1,48-2,22 TBq/mmole) pendant 1 heure à 37°C. Une post-incubation d'une heure suit dans un Krebs-Henseleit dépourvu de [³H]-thymidine. Après lavage dans un tampon Tris-EDTA (Tris: 10 mM; EDTA: 10 mM; NaCl: 100 mM) l'aorte est stockée à -20°C. Après décongélation, l'intima-média est prélevée, broyée et incubée pendant 30 minutes en présence de pronase (type B, nuclease free, à 4 mg/ml) et de SDS (5 mg/ml). L'homogénat est centrifugé à 2250 g pendant 10 minutes à 10°C et le surnageant est divisé en 2 fractions, l'une pour le dosage de [³H]-ADN, l'autre pour le dosage de l'ADN total.

Pour le dosage de [³H]-ADN, l'ADN est coprécipité par addition de 13,3 µl de BSA (3 mg/ml de Tris-EDTA) et de 1 ml d'acide trichloracétique à 20 % par ml de surnageant. Après 30 minutes à 4°C, le précipité est filtré sur une membrane en nitrocellulose (porosité : 45 µm), et la radioactivité est déterminée par un spectrofluorimètre à scintillation liquide. La détermination de la quantité d'ADN total est effectuée par spectrofluorimétrie en utilisant comme sonde fluorescente le DAPI (4',6-diamidino-2-phénylindole) (100 ng/ml) qui s'intercale entres les bases A et T de l'ADN. La longueur d'onde d'excitation est de 360 nm et celle d'émission de 450nm. La concentration d'ADN est déterminée par rapport à une gamme d'étalon d'ADN, et la quantité d'ADN contenue dans l'aorte est ensuite calculée ainsi que le rapport cpm/µg d'ADN correspondant à l'index de prolifération.

Les résultats sont reproduits dans le tableau II :

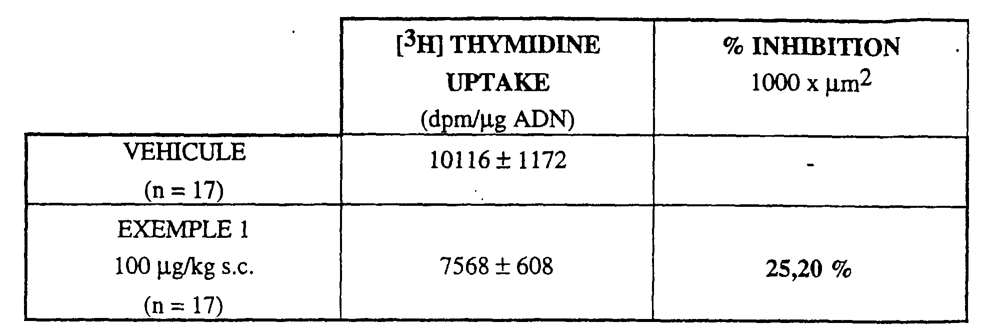

Durée du traitement : de -2 à +3 jours après la lésion. (Durée totale : 5 jours)
Mesures effectuées 3 jours après la lésion sur la carotide de rat

### COMPOSITION PHARMACEUTIQUE

### EXEMPLE 9 : Comprimé : formule de préparation pour 1000 comprimés dosés à 2 mg.

| | |
|---|---|
| Composé de l'exemple 1 | 2g |
| Hydroxypropyl cellulose | 2g |
| Amidon de blé | 10g |
| Lactose | 100g |
| Stéarate de magnésium | 3g |
| Tale | 3g |

## Revendications

1. Composés de formule (I) 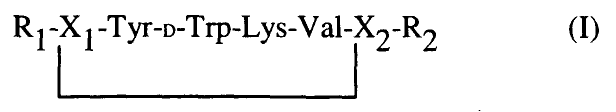 dans laquelle :
- R₁ représente un résidu d'acide aminé, de configuration L ou D, acétylé ou non acétylé,
- R₂ représente un résidu d'acide aminé, de configuration L ou D, possédant une fonction C-terminale amide ou acide libre,
- X₁ et X₂ sont choisis parmi des résidus d'acides aminés permettant une cyclisation par une liaison amide et conférant au cycle ainsi formé un nombre d'atomes égal à 22,
- Tyr représente le résidu _{L}-Tyrosyle,
- _{D}-Trp représente le résidu _{D}-Tryptophyle,
- Lys représente le résidu _{L}-Lysyle,
- Val représente le résidu _{L}-Valyle,
leurs énantiomères, diastéréoisomères et épimères, ainsi que leurs sels d'addition à un acide ou à une base pharmaceutiquement acceptable.

2. Composés selon la revendication 1 dans lesquels X₁ représente le résidu de l'acide _{L}-glutamique (Glu) et X₂ le résidu de l'acide _{L}-2,4-diaminobutyrique (Dab), leurs énantiomères, diastéréoisomères et épimères, ainsi que leurs sels d'addition à un acide ou à une base pharmaceutiquement acceptable.

3. Composés selon la revendication 1 dans lesquels X₁ représente le résidu de l'acide _{L}-aspartique (Asp) et X₂ le résidu de la _{L}-ornithine (Orn), leurs énantiomères, diastéréoisomères et épimères, ainsi que leurs sels d'addition à un acide ou à une base pharmaceutiquement acceptable.

4. Composés selon la revendication 1 dans lesquels X₁ représente le résidu de l'acide _{L}-homoglutamique (*homo* Glu) et X₂ le résidu de l'acide _{L}-2,3-diaminopropionique (Dpr), leurs énantiomères, diastéréoisomères et épimères, ainsi que leurs sels d'addition à un acide ou à une base pharmaceutiquement acceptable.

5. Composés selon la revendication 1 dans lesquels X₁ représente le résidu de l'acide _{L}-2,4-diaminobutyrique (Dab) et X₂ le résidu de l'acide glutamique (Glu), leurs énantiomères, diastéréoisomères et épimères, ainsi que leurs sels d'addition à un acide ou à une base pharmaceutiquement acceptable.

6. Composés selon la revendication 1 dans lesquels X₁ représente le résidu de la _{L}-ornithine (Orn) et X₂ le résidu de l'acide _{L}-aspartique (Asp), leurs énantiomères, diastéréoisomères et épimères, ainsi que leurs sels d'addition à un acide ou à une base pharmaceutiquement acceptable.

7. Composés selon la revendication 1 dans lesquels X₁ représente le résidu de l'acide _{L}-2,3-diaminopropionique (Dpr) et X₂ le résidu de l'acide _{L}-homoglutamique (*homo* Glu), leurs énantiomères, diastéréoisomères et épimères, ainsi que leurs sels d'addition à un acide ou à une base pharmaceutiquement acceptable.

8. Composé selon la revendication 1 qui est le : 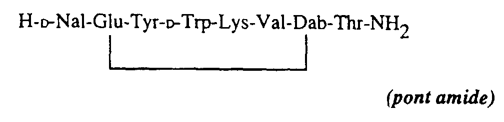 ses énantiomères, diastéréoisomères et épimères, ainsi que ses sels d'addition à un acide ou une base pharmaceutiquement acceptable.

9. Procédé de préparation des composés de formule (I) selon la revendication 1 caractérisé en ce qu'il est effectué sur un automate qui effectue de façon répétitive et programmable des cycles de déprotections, couplages et lavages nécessaires à l'introduction séquentielle des acides aminés dans la chaîne peptidique, l'acide aminé, de préférence C-terminal, étant fixé sur une résine conventionnellement utilisée pour la préparation de polypeptides, le choix de la résine permettant la fixation de la fonction C-terminale souhaitée, en introduisant les acides aminés en excès, par rapport au degré de substitution de la résine et en quantité environ équivalente par rapport aux agents de couplage, un à un, dans l'ordre déterminé par l'opérateur, chaque cycle de synthèse correspondant à l'introduction d'un acide aminé comportant une déprotection, des lavages successifs destinés à éliminer les réactifs, un couplage avec activation de l'acide aminé et de nouveaux lavages, chacune de ces opérations étant suivie d'une filtration réalisée sur verre fritté incorporé au réacteur dans lequel s'effectue la synthèse, puis, les cycles de déprotections, couplages et lavages étant terminés, par traitement par un acide fort en présence d'anisole, afin de séparer le peptide de la résine et de libérer éventuellement le peptide de ses groupes protecteurs, les composés de formule (I) ainsi obtenus sont alors purifiés par des techniques classiques de purification.

10. Compositions pharmaceutiques contenant comme principe actif au moins un composé selon l'une quelconque des revendications 1 à 8, seul ou en combinaison avec un ou plusieurs excipients ou véhicules inertes, non toxiques, pharmaceutiquement acceptables.

11. Compositions pharmaceutiques selon la revendication 10 contenant au moins un principe actif selon l'une quelconque des revendications 1 à 8, utiles, par leur activité inhibitrice sur la composante de prolifération des cellules musculaires lisses vasculaires, dans le traitement et la prévention des lésions vasculaires athéroscléreuses, dans la prévention des resténoses vasculaires après pontage, dilatation vasculaire, et après transplantation cardiaque et utiles dans la prévention et le traitement des altérations vasculaires liées à l'hypertension artérielle, au diabète ainsi que dans le traitement de cancers et de maladies dermatologiques.

## Patentansprüche

1. Verbindungen der Formel (I) 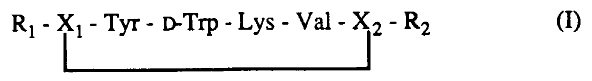 in der
- R₁ einen Aminosäurerest in der Konfiguration L oder D, der acetyliert oder nicht acetyliert ist.
- R₂ einen Aminosäurerest in der Konfiguration L oder D, der eine C-terminale Amidfunktion oder freie Säurefunktion aufweist,
- X₁ und X₂ ausgewählt sind aus Aminosäureresten, die eine Cyclisierung über eine Amidbindung ermöglichen und in dieser Weise einen Ring mit 22 Atomen ergeben,
- Tyr den L-Tyrosylrest,
- D-Trp den D-Tryptophylrest,
- Lys den L-Lysylrest,
- Val den L-Valylrest
bedeuten,
deren Enantiomere, Diastereoisomere und Epimere sowie deren Additionssalze mit einer pharmazeutisch annehmbaren Säure oder Base.

2. Verbindungen nach Anspruch 1, worin X₁ den Rest der L-Glutaminsäure (Glu) und X₂ den Rest der L-2,4-Diaminobuttersäure (Dab) bedeuten, deren Enantiomere, Diastereoisomere und Epimere sowie deren Additionssalze mit einer pharmazeutisch annehmbaren Säure oder Base.

3. Verbindungen nach Anspruch 1, worin X₁ den Rest der L-Asparaginsäure (Asp) und X₂ den Rest von L-Ornithin (Orn) bedeuten, deren Enantiomere, Diastereoisomere und Epimere sowie deren Additionssalze mit einer pharmazeutisch annehmbaren Säure oder Base.

4. Verbindungen nach Anspruch 1, worin X₁ den Rest der L-Homoglutaminsäure *(homo* Glu) und X₂ den Rest der L-2,3-Diaminopropionsäure (Dpr) bedeuten, deren Enantiomere, Diastereoisomere und Epimere sowie deren Additionssalze mit einer pharmazeutisch annehmbaren Säure oder Base.

5. Verbindungen nach Anspruch 1, worin X₁ den Rest der L-2,4-Diaminobuttersäure (Dab) und X₂ den Rest der Glutaminsäure (Glu) bedeuten, deren Enantiomere, Diastereoisomere und Epimere sowie deren Additionssalze mit einer pharmazeutisch annehmbaren Säure oder Base.

6. Verbindungen nach Anspruch 1, worin X₁ den Rest von L-Ornithin (Orn) und X₂ den Rest der L-Asparaginsäure (Asp) bedeuten, deren Enantiomere, Diastereoisomere und Epimere sowie deren Additionssalze mit einer pharmazeutisch annehmbaren Säure oder Base.

7. Verbindungen nach Anspruch 1, worin X₁ den Rest der L-2,3-Diaminopropionsäure (Dpr) und X₂ den Rest der L-Homoglutaminsäure (*homo* Glu) bedeuten, deren Enantiomere, Diastereoisomere und Epimere sowie deren Additionssalze mit einer pharmazeutisch annehmbaren Säure oder Base.

8. Verbindung nach Anspruch 1, nämlich: 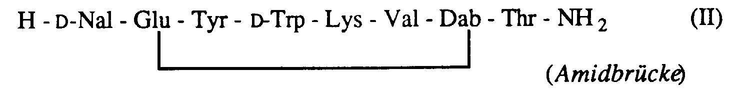 deren Enantiomere, Diastereoisomere und Epimere sowie deren Additionssalze mit einer pharmazeutisch annehmbaren Säure oder Base.

9. Verfahren zur Herstellung der Verbindungen der Formel (I) nach Anspruch 1, **dadurch gekennzeichnet,** daß es mit einem Automaten durchgeführt wird, der in wiederholter und programmierbarer Weise Schutzgruppenabspaltungs-, Kupplungs- und Wasch-Zyklen durchführt, die zur sequentiellen Einführung von Aminosäuren in die Peptidkette erforderlich sind, wobei die vorzugsweise C-terminale Aminosäure an ein üblicherweise für die Herstellung von Polypeptiden verwendetes Harz gebunden ist, die Auswahl des Harzes die Bindung der gewünschten C-terminalen Funktion ermöglicht, wobei die Aminosäuren im Überschub in bezug auf den Substitutionsgrad des Harzes und in etwa äquivalenter Menge in bezug auf die Kupplungsmittel eine nach der anderen in von dem Operator bestimmten Reihenfolge zugeführt werden, jeder Synthesezyklus der Einführung einer Aminosäure entspricht und eine Schutzgruppenabspaltung, aufeinanderfolgende Waschungen zur Entfernung der Reagenzien, eine Kupplung mit Aktivierung der Aminosäure und erneute Waschvorgänge umfaßt, wobei jeder dieser Vorgänge von einer Filtration über eine Glasfritte, welche in dem Reaktor, in dem die Synthese durchgeführt wird, angeordnet ist, und dann, wenn die Schutzgruppenabspaltungs-, Kupplungs- und Wasch-Zyklen beendet sind, der Behandlung mit einer starken Säure in Gegenwart von Anisol zurAbtrennung des Peptids von dem Harz und der eventuellen Befreiung des Peptids von seinen Schutzgruppen und anschließend der Cyclisierung des Peptids in Gegenwart von BOP und von DIPEA gefolgt wird, wobei die in diese Weise erhaltenen Verbindungen der Formel (I) mit Hilfe klassischer Reinigungsverfahren gereinigt werden.

10. Pharmazeutische Zubereitungen enthaltend als Wirkstoff mindestens eine Verbindung nach einem der Ansprüche 1 bis 8 allein oder in Kombination mit einem oder mehreren inerten, nichttoxischen, pharmazeutisch annehmbaren Trägermaterialien oder Bindemitteln.

11. Pharmazeutische Zubereitungen nach Anspruch 10, enthaltend mindestens einen Wirkstoff nach einem der Ansprüche 1 bis 8, welche aufgrund ihrer inhibierenden Wirkung auf die Komponente der Vermehrung von glatten Gefäßmuskelzellen bei der Behandlung und Vorbeugung von atherosklerotischen Gefäßerkrankungen, bei der Vorbeugung von Gefäßrestenosen nach der Bypassbildung, der Gefäßerweiterung und nach der Herztransplantation verwendet werden können und für die Vorbeugung und die Behandlung von Gefäßveränderungen, die mit einer arteriellen Hypertension, dem Diabetes verknüpft sind sowie bei der Behandlung von Krebsen und dermatologischen Erkrankungen geeignet sind.

## Claims

1. Compounds of formula (I) 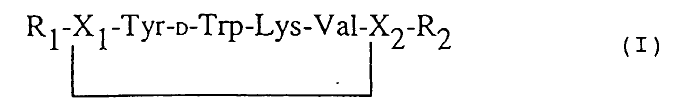 in which:
- R₁ represents an acetylated or non-acetylated amino acid residue having the L or D configuration,
- R₂ represents an amino acid residue having the L or D configuration, the C-terminal function of which is an amide or free acid function,
- X₁ and X₂ are selected from amino acid residues that permit cyclisation by means of an amide bond and that confer on the ring so formed a number of atoms equal to 22,
- Tyr represents the L-tyrosyl residue,
- D-Trp represents the D-tryptophyl residue,
- Lys represents the L-lysyl residue, and
- Val represents the L-valyl residue,
their enantiomers, diastereoisomers and epimers, as well as their addition salts with a pharmaceutically acceptable acid or base.

2. Compounds according to claim 1 in which X₁ represents the L-glutamic acid residue (Glu) and X₂ represents the L-2,4-diaminobutyric acid residue (Dab), their enantiomers, diastereoisomers and epimers, as well as their addition salts with a pharmaceutically acceptable acid or base.

3. Compounds according to claim 1 in which X₁ represents the L-aspartic acid residue (Asp) and X₂ represents the L-ornithine residue (Orn), their enantiomers, diastereoisomers and epimers, as well as their addition salts with a pharmaceutically acceptable acid or base.

4. Compounds according to claim 1 in which X₁ represents the L-homoglutamic acid residue (homoGlu) and X₂ represents the L-2,3-diaminopropionic acid residue (Dpr), their enantiomers, diastereoisomers and epimers, as well as their addition salts with a pharmaceutically acceptable acid or base.

5. Compounds according to claim 1 in which X₁ represents the L-2,4-diaminobutyric acid residue (Dab) and X₂ represents the glutamic acid residue (Glu), their enantiomers, diastereoisomers and epimers, as well as their addition salts with a pharmaceutically acceptable acid or base.

6. Compounds according to claim 1 in which X₁ represents the L-ornithine residue (Orn) and X₂ represents the L-aspartic acid residue (Asp), their enantiomers, diastereoisomers and epimers, as well as their addition salts with a pharmaceutically acceptable acid or base.

7. Compounds according to claim 1 in which X₁ represents the L-2,3-diaminopropionic acid residue (Dpr) and X₂ represents the L-homoglutamic acid residue (homoGlu), their enantiomers, diastereoisomers and epimers, as well as their addition salts with a pharmaceutically acceptable acid or base.

8. Compound according to claim 1 which is: 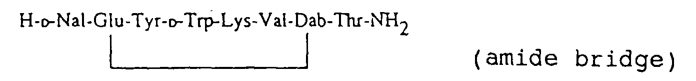 its enantiomers, diastereoisomers and epimers, as well as its addition salts with a pharmaceutically acceptable acid or base.

9. Process for the preparation of compounds of formula (I) according to claim 1, characterised in that it is effected using an automatic apparatus which carries out, in a repetitive and programmable manner, cycles of deprotections, couplings and washing steps which are necessary for the sequential introduction of the amino acids into the peptide chain, the amino acid, which is preferably C-terminal, being attached to a resin customarily used for the preparation of polypeptides, the choice of resin allowing the desired C-terminal function to be attached, by introducing the amino acids in excess, relative to the degree of substitution of the resin, and in an approximately equivalent amount relative to the coupling agents, one by one, in the order determined by the operator, each synthesis cycle corresponding to the introduction of one amino acid comprising deprotection, successive washing steps in order to remove the reagents, coupling with activation of the amino acid, and further washing steps, each of those operations being followed by filtration which is carried out through sintered glass incorporated into the reactor in which the synthesis is carried out, then, once the cycles of deprotections, couplings and washing steps are complete, by treatment with a strong acid in the presence of anisole in order to separate the peptide from the resin and, where appropriate, to free the peptide of its protecting groups, and then cyclisation of the peptide in the presence of BOP and DIPEA, the compounds of formula (I) so obtained then being purified by conventional purification techniques.

10. Pharmaceutical compositions containing as active ingredient at least one compound according to any one of claims 1 to 8, on its own or in combination with one or more inert, non-toxic, pharmaceutically acceptable excipients or carriers.

11. Pharmaceutical compositions according to claim 10 containing at least one active ingredient according to any one of claims 1 to 8, for use, on account of their inhibiting activity on the proliferation component of vascular smooth muscle cells, in the treatment and prevention of atherosclerotic vascular lesions, in the prevention of vascular re-stenoses following bypass surgery, following vascular dilation, and following heart transplant, and for use in the prevention and treatment of vascular changes associated with arterial hypertension or with diabetes, as well as in the treatment of cancers and dermatological disorders.
